Europäisches Patentamt

European Patent Office

Office européen des brevets

(11) Publication number: **0 445 750 A1**

# EUROPEAN PATENT APPLICATION

(21) Application number: 91103351.2

(22) Date of filing: 06.03.91

(51) Int. Cl.⁵: **C12P 21/08**, C12Q 1/48, G01N 33/577

(30) Priority: 06.03.90 JP 54567/90

(43) Date of publication of application:
11.09.91 Bulletin 91/37

(84) Designated Contracting States:
DE FR GB

(71) Applicant: KONICA CORPORATION
26-2, Nishishinjuku 1-chome, Shinjuku-ku
Tokyo 160(JP)

(72) Inventor: Uemura, Morito
c/o Konica Corporation, 1-Sakuramachi
Hino-shi, Tokyo 191(JP)
Inventor: Yamasaki, Masahiko
c/o Konica Corporation, 1-Sakuramachi
Hino-shi, Tokyo 191(JP)
Inventor: Uejima, Takao
c/o Konica Corporation, 1-Sakuramachi
Hino-shi, Tokyo 191(JP)
Inventor: Sakaguchi, Takashi
c/o Konica Corporation, 1-Sakuramachi
Hino-shi, Tokyo 191(JP)

(74) Representative: Türk, Gille, Hrabal
Brucknerstrasse 20
W-4000 Düsseldorf 13(DE)

(54) Anti-cancer-related human galactosyltransferase monoclonal antibody.

(57) A monoclonal antibody which may be used as a diagnostic reagent of cancer is disclosed. The monoclonal antibody of the present invention specifically reacts with a cancer-related human galactosyltransferase having a molecular weight of about 50,000 determined by SDS-polyacrylamide gel electrophoresis under reduced condition, the galactosyltransferase having a reactivity with MAb4880 (FERM BP-1758) under native conditions, a part of which self-associates, the reactivity of the galactosyltransferase with MAb4880 and the degree of self-association of the galactosyltransferase being promoted when the galactosyltransferase is heated under reducing conditions so that a part of the galactosyltransferase being detected in a fraction of a molecular weight of not less than 200,000 in gel permeation chromatography.

EP 0 445 750 A1

## BACKGROUND OF THE INVENTION

I. Field of the Invention

The present invention relates to a monoclonal antibody which is specific to a cancer-related human galactosyltransferase. The present invention also relates to a hybridoma producing the monoclonal antibody and to a method of measuring the cancer-related human galactosyltransferase using the monoclonal antibody of the present invention. The monoclonal antibody of the present invention may be used as a diagnostic reagent of cancer.

II. Description of the Related Art

Galactosyltransferase (hereinafter also referred to as "GT" for short) is an enzyme which catalyzes the transfer of galactose from uridine-5'-diphosphogalactose (UDP-galactose) to non-reducing terminals of oligosaccharides of various glycoproteins or monosaccharides, which occurs in almost all the tissues in the body. Abnormal activity of GT has been recognized in various malignant tumors and the possibility of employing the GT as a cancer marker has been investigated. As a result, it was discovered that the level of GT-II which is an isozyme of GT in serum has a close relationship with the existence of a cancer. The GT-II is defined as the GT enzyme activity in a band obtained in Native-PAGE (polyacrylamide gel electrophoresis), which has slower mobility than the GT-I which mainly exists in normal sera, as described in Biochem. Biophys. Res. Common, 65(2), pp.545-551, 1975.

The present inventors made further study to find that a GT specifically related to cancer exists, that the GT-II band recognized in the Native-PACE is only a phenomenon caused by the cancer-related GT and that the cancer-related GT is also contained in the origin of the Native-PACE (U.S. Serial No. 07/462,096 filed January 8, 1990). The cancer-related GT exhibits the behavior of GT-II and has the following essential properties. That is, the cancer-related GT is a galactosyltransferase which a molecular weight of about 50,000 determined by SDS-polyacrylamide gel electrophoresis under reduced condition, which has a reactivity with MAb4880 (FERM BP-1758) under native conditions, a part of which self-associates, of which reactivity with MAb4880 and the degree of self-association are promoted when it is heated under reducing conditions so that a part of which is detected in a fraction of a molecular weight of not less than 200,000 in gel permeation chromatography.

As mentioned above, a GT which is related to cancer has been isolated and it has been shown that the cancer-related GT is useful as a cancer marker. Thus, if a monoclonal antibody specific to the cancer-related GT exists, the monoclonal antibody may be used as a diagnostic reagent of cancer.

## SUMMARY OF THE INVENTION

Accordingly, an object of the present invention is to provide a novel monoclonal antibody which specifically reacts with the cancer-related GT.

That is, the present invention provides a monoclonal antibody which specifically reacts with a cancer-related human galactosyltransferase, said galactosyltransferase having a molecular weight of about 50,000 determined by SDS-polyacrylamide gel electrophoresis under reduced condition, said galactosyltransferase having a reactivity with MAb4880 (FERM BP-1758) under native conditions, a part of said galactosyltransferase self-associating, the reactivity of said galactosyltransferase with MAb4880 and the degree of self-association of said galactosyltransferase being promoted when said galactosyltransferase is heated under reducing conditions so that a part of said galactosyltransferase being detected in a fraction of a molecular weight of not less than 200,000 in gel permeation chromatography.

The present invention also provides a hybridoma which produces the monoclonal antibody of the present invention.

The present invention still further provides a method of measuring cancer-related human galactosyltransferase in a sample comprising specifically reacting the monoclonal antibody of the present invention with the cancer-related human galactosyltransferase possibly contained in said sample.

By the present invention, a novel monoclonal antibody which specifically recognizes the cancer-related human galactosyltransferase, as well as a hybridoma producing the monoclonal antibody and a method of measuring the cancer-related human galactosyltransferase using the monoclonal antibody was provided. By the present invention, the cancer-related human galactosyltransferase in a sample may be measured, so that the present invention contributes to the diagnosis of cancer such as ovarian cancer.

## BRIEF DESCRIPTION OF THE DRAWING

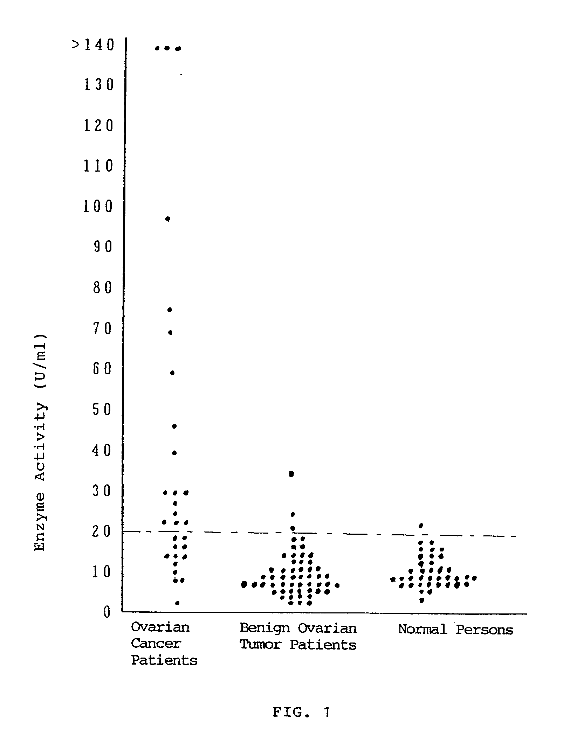

Fig. 1 shows the results of the measurements of the cancer-related human galactosyltransferase in sera from ovarian cancer patients, benign ovarian tumor patients and from normal persons.

## DETAILED DESCRIPTION OF THE PREFERRED EMBODIMENTS

As described above, the monoclonal antibody of the present invention specifically reacts with the above-described cancer-related GT. The cancer-related GT is known and described in Japanese Laid Open Patent Application (Kokai) No. 2-186984 or in U.S. copending patent application serial No. 07/462,096 mentioned above. The MAb4880 employed in the definition of the cancer-related GT is a monoclonal antibody whose corresponding antigen is GT purified from ascites of a patient suffering from ovarian cancer. The MAb4880 is prepared by the same manner as described in Cancer Research, vol. 48, p.5325, 1988, and disclosed in Japanese Laid Open Patent Application (Kokai) No. 174,100/87. A hybridoma producing the MAb4880 is deposited with Fermentation Research Institute in Japan in accordance with the Budapest Treaty under an accession number of FERM BP-1758.

The monoclonal antibody of the present invention may be prepared by the so called hybridoma method using the cancer-related GT as the immunogen. The process of preparing the monoclonal antibody of the present invention will now be described in detail.

The immunogen used in the preparation of the monoclonal antibody of the present invention may be purified from ascites of a cancer patient, e.g., a patient suffering from ovarian cancer, by α-lactoalbumin affinity chromatography in accordance with the method described in Cancer Research, vol. 48, p.5325, 1988 in the same manner as the normal human GT is purified, as described in detail in the examples hereinbelow described. It should be noted, however, the cancer-related GT exists not only in the patients suffering from ovarian cancer but also in the patients suffering from other cancers such as bladder cancer, colorectal cancer, lung cancer, pancreas cancer, esophagus cancer and liver cancer. Further, the cancer-related GT occurs not only in ascites but also other body fluids such as serum.

A mammal such as mouse or rat is immunized with the immunogen. The immunization may be carried out by a conventional method. For example, the immunogen may be administered intraperitoneally or intravenously to an animal together with an adjuvant such as Freund's complete adjuvant.

Then antibody-producing cells such as spleen cells are collected from the immunized animal and the cells are fused with, for example, mouse myeloma cells. As the myeloma cells, various known myeloma cells such as X63-Ag 8.653 may be employed. As the fusing agent, polyethylene glycol may be employed. The mixing ratio of the antibody-producing cells to the myeloma cells may preferably be 1:1 to 10:1 in terms of the number of the cells.

After the cell fusion, by culturing the fused cells in a conventional selection medium such as HAT medium, hybridomas can be selected. Since the myeloma cells cannot be alive in HAT medium, the selection of the hybridomas can be attained by simply selecting the cells which grow in HAT medium.

After the colonies of the hybridomas sufficiently grew, the screening of the hybridoma producing the desired monoclonal antibody and the cloning of the hybridoma are carried out. The screening of the hybridoma may be carried out by a conventional method such as ELISA. The hybridomas producing a monoclonal antibody which reacts with the immunogen and which does not have cross-reactivity with serum proteins are selected and then cloned by the limiting dilution method. By this process, a desired cloned hybridoma producing the monoclonal antibody of the present invention may be obtained.

The monoclonal antibody of the present invention may be recovered by culturing the desired hybridoma in a culturing medium and separating the monoclonal antibody from the supernatant of the culture medium or by intraperitoneally administering the desired hybridoma to mice and recovering the monoclonal antibody from the ascites of the mice. Further, if desired, the monoclonal antibody of the present invention may be purified by conventional methods such as precipitation with ammonium sulfate, gel permeation chromatography and ion-exchange chromatography.

As will be concretely described in the examples below, by the above-described process, three monoclonal antibodies of the present invention, that is, monoclonal antibodies named MAb7907, MAb8513 and MAb8677 were obtained. These monoclonal antibodies have been deposited with Fermentation Research Institute of Japan under the Budapest Treaty, the accession numbers being given in the examples hereinbelow described.

The measurement of the cancer-related GT in a sample using the monoclonal antibody of the present invention may be accomplished according to a conventional immunoassay utilizing the specific antigen-antibody reaction. For example, the immunoassay may be carried out according to the sandwich assay which per se is well-known in the art. In the sandwich assay, the monoclonal antibody of the present invention may be fixed to an appropriate solid carrier such as microtiterplate or plastic beads and the fixed monoclonal antibody is made to contact the sample. Thereafter, a second antibody whose corresponding epitope is different from the that of the monoclonal antibody of the present invention, which second antibody is labelled with an appropriate marker such as a radioisotope (such as $^{125}I$) or peroxidase and then the solid carriers are washed, followed by measurement of the second antibody according to the marker attached thereto. In this case, as the second antibody, MAb4880 as well as monoclonal antibodies MAb8507 and MAb8628 may preferably be employed.

In cases where the monoclonal antibody of the present invention is used for diagnosis of cancer, body fluids, especially serum, may preferably be used as the sample.

It has been found that the monoclonal antibody of the present invention exhibits high specificity to the cancer-related GT in self-associated state, while most of the cancer-related GT molecules exist in the form of dissociated molecules with low molecular weight in serum. However, when the monoclonal antibody of the present invention is used in the fixed state, by carrying out the immunological reaction at a relatively high temperature, that is, preferably at 37 - 56°C, the reactivity of the immobilized monoclonal antibody with the cancer-related GT may be promoted. This is presumably because that the three dimensional structure of the cancer-related GT is changed so that its reactivity with the monoclonal antibody is promoted.

[Examples]

The present invention will now be described by way of examples thereof. It should be noted that the examples are presented for the illustration purpose only and should not be interpreted in any restrictive way.

Example 1

Preparation of Immunogen

From 1 liter of ascites from a patient suffering from ovarian cancer, GT was purified by α-lactoalbumin affinity chromatography in accordance with the description in Cancer Research, vol. 48, p.5325, 1988. More particularly, the ascites was dialyzed overnight against purified water at 4°C and the insoluble mass was removed by centrifugation. To the resulting solution, Tris-buffer (pH 7.2), manganese chloride (MnCl₂) and N-acetylglucosamine were added to attain the final level of 20 mM, 10 mM and 5 mM, respectively, and the resulting mixture was applied to an α-lactoalbumin affinity column (2.5 cm x 50 cm). After the column was washed with 1 liter of the same buffer as mentioned above containing manganese chloride and N-glucosamine, elution was carried out with the same buffer but not containing N-acetylglucosamine to obtain GT fractions. The same chromatography operation as described above was repeated for the obtained GT fractions to further purify the GT.

In 1.0 ml of phosphate buffered saline (PBS), 0.5 mg of the thus obtained cancer-related GT was dissolved and this solution was used as the immunogen.

Immunization and Cell Fusion

To a Balb/c mouse (female, 6 weeks old), 0.2 ml of a uniform mixture of 0.5 ml of the immunogen described above and 0.5 ml of Freund's complete adjuvant was intraperitoneally administered. Three weeks after the first immunization, 0.2 ml of the same mixture as used in the first immunization except that Freund's incomplete adjuvant was used in place of the Freund's complete adjuvant was intraperitoneally administered. Two weeks after the second immunization, 100 μg of the immunogen alone was injected in a tail vein.

Three days after the final immunization, spleen cells of the mouse were taken and washed with RPMI 1640 medium. A cell suspension containing $4 \times 10^8$ spleen cells and a cell suspension containing $8 \times 10^7$ mouse myeloma cells (X63-Ag8.653) were mixed and the medium was removed by centrifugation. In a water bath at 37°C, 1 ml of polyethylene glycol-RPMI 1640 medium was gradually added and the cell mixture was gently stirred so as to allow cell fusion. The medium was then removed by centrifugation and 40 ml of RPMI 1640 medium containing 15% by weight of fetal calf serum (FCS) was added. The resulting mixture was placed in the wells of a 96-well microplate in the amount of 0.15 ml/well. On the next day, 0.15 ml of HAT medium (RPMI 1640 medium containing $4 \times 10^{-7}M$ aminoputerin, $1.6 \times 10^{-5}M$ thymidine, $1 \times 10^{-4}M$ hypoxanthine and 10% by weight of FCS) was added to the each well. Half volume of the medium in each well was replaced with fresh HAT medium every 3 or 4 days. After 2 weeks from the beginning of the culture, the growth of hybridomas was observed in 80% of the wells.

Selection of Hybridomas

The screening of the monoclonal antibody contained in the supernatant of the hybridoma culture medium was carried out by ELISA using the cancer-related GT obtained above as an antigen.

More particularly, 2 $\mu$ g/ml of the antigen in PBS was adsorbed to wells in a microtiter plate for ELISA and the wells were blocked with 1% BSA in PBS. The culture media of the hybridomas were separately placed in the wells in the microtiter plate for ELISA so as to allow antibody-antigen reaction. Goat anti-mouse immunoglobulin antibody labelled with peroxidase was then reacted with the reaction product and o-phenylenediamine which is a substrate of peroxidase was added to the wells, followed by measurement of absorbance at 492 nm. As a result, 8 hybridomas which react with the cancer-related GT and which do not react with serum proteins were obtained in 14 times run of the cell fusion process.

The thus obtained hybridomas were transferred to HT medium (HAT medium from which aminoputerin is removed), and then transferred to RPMI 1640 medium containing 10% by weight of FCS.

The thus obtained hybridomas were then cloned by the limiting dilution method. That is, cell suspensions of the hybridomas were placed in wells of a microplate after sufficient dilution so as to attain a cell population of 0.5 - 4 cells per well. The hybridomas were cultured with 1 x $10^6$/well of mouse thymus cells. After two weeks from the beginning of this culture, the hybridomas producing the desired monoclonal antibody were selected by the above-described ELISA.

The above-described operation was repeated to obtain stable hybridomas MAb7907, MAb8513, MAb8677, MAb8628, MAb8507, MAb8611, MAb8913 and MAb8919 were established.

By the conventional ELISA, the class of the thus obtained monoclonal antibodies was determined. As a result, monoclonal antibodies MAb7907 and MAb8513 were classified as IgM, MAb8628, MAb8677, MAb8507, MAb8611 and MAb8919 were classified as IgG$_1$, MAb8913 was classified as IgG$_2$b. Hybridomas MAb7907, MAb8513, MAb8628 and MAb8677 were deposited with Fermentation Research Institute of Japan under the Budapest Treaty under accession numbers of FERM BP-3279, FERM BP-3278, FERM BP-3280 and FERM BP-3281, respectively.

Example 2

Analysis of Epitope of Antigen

The purified antigen obtained in Example 1 was dissolved in PBS to a concentration of 1 $\mu$ g/ml and the resulting solution was adsorbed in the wells of a microtiter plate. The wells were washed with PBS and blocked with 1% by weight of BSA. Monoclonal antibodies MAb4880 and MAb7907 were labelled with horse radish peroxidase (HRP) by the conventional periodic acid method (The monoclonal antibodies labelled with HRP are hereinafter expressed as MAb4880-HRP, MAb7907-HRP or the like. Further, the monoclonal antibodies labelled with HRP are collectively referred to as "labelled body"), and the thus obtained labelled bodies were 2000-fold diluted with 1 wt% BSA solution. To each well, 100 $\mu$l of the resulting labelled body solution was added and the reaction was allowed to occur at 37°C for 1 hour. After the reaction, the wells were washed with PBS and o-phenylenediamine was added to the wells, followed by the measurement of the absorbance at 492 nm of the solution in each well. On the other hand, to MAb4880-HRP or MAb7907-HRP, the monoclonal antibodies shown in Table 1 were added to a final concentration of 10 $\mu$ g/ml and the same operation as described above was repeated so as to observe the influence by the addition of the monoclonal antibodies. The measured absorbance is shown in Table 1.

EP 0 445 750 A1

T a b l e 1

| MAb Admixed with Labelled Body | Labelled Body MAb4880-HRP | Labelled Body MAb7907-HRP |
|---|---|---|
| 4 8 8 0 | 0.709 | > 2.000 |
| 7 9 0 7 | > 2.000 | 0.192 |
| 8 5 0 7 | 0.235 | > 2.000 |
| 8 5 1 3 | > 2.000 | 0.013 |
| 8 6 1 1 | > 2.000 | > 2.000 |
| 8 6 2 8 | 0.059 | > 2.000 |
| 8 6 7 7 | > 2.000 | 0.112 |
| 8 9 1 3 | > 2.000 | > 2.000 |
| 8 9 1 9 | > 2.000 | > 2.000 |
| 1%B S A | > 2.000 | > 2.000 |

From the results shown in Table 1, the monoclonal antibodies may be classified into three groups as follows:

Group 1:     MAb4880, MAb8507 and MAb8628
Group 2:     MAb7907, MAb8513 and MAb8677
Group 3:     MAb8611, MAb8913 and MAb8919

It is expected that when a sandwich assay is carried out, combinations of the monoclonal antibodies belonging to the same group should be avoided. Further, as described in Example 3 below, the monoclonal antibodies of the present invention are those belonging to Group 2. When carrying out a sandwich assay, it is preferred to fix the monoclonal antibody of the present invention belonging to Group 2 to a solid carrier and to use the monoclonal antibody belonging to Group 1 as the second antibody.

Example 3

Biotin was attached to the antigen obtained in Example 1 and was fractioned by gel permeation chromatography Sup-12 FPLC (commercially available from Pharmacia) (solvent: PBS; flow rate: 0.5

6

ml/min). The fraction of the molecular weight of from about 500,000 to about 2,000,000 was named P-1, and the fraction of the molecular weight of about 50,000 to about 300,000 was named P-2. The various monoclonal antibodies at a concentration of 10 $\mu$g/ml shown in Table 2 were fixed to the wells in a microtiter plate. After blocking the wells with 1% BSA, P-1 or P-2 diluted with 1 wt% of BSA to a prescribed concentration was added to each well in the amount of 100 $\mu$l/well and the reaction was allowed to occur at 37°C for 1 hour. After the reaction, the wells were washed with PBS and streptoavidin-HRP 2000-fold diluted with 1 wt% BSA was added to each well in the amount of 100 $\mu$l/well. The reaction was allowed to occur at room temperature for 30 minutes. After the reaction, the wells were washed with PBS and o-phenylenediamine was added to the wells, followed by the measurement of absorbance at 492 nm. The results are shown in Table 2.

## Table 2

| Immobilized MAb | P − 1 | | | P − 2 | | |
|---|---|---|---|---|---|---|
| | 1:100 | 1:300 | 1:900 | 1:100 | 1:300 | 1:900 |
| 4 8 8 0 | >2.000 | 0.615 | 0.207 | 0.451 | 0.171 | 0.062 |
| 7 9 0 7 | 0.751 | 0.380 | 0.144 | 0.066 | 0.034 | 0.015 |
| 8 5 0 7 | 1.860 | 0.603 | 0.193 | 0.366 | 0.160 | 0.052 |
| 8 5 1 3 | 0.687 | 0.227 | 0.079 | 0.063 | 0.033 | 0.019 |
| 8 6 1 1 | 0.100 | 0.028 | 0.013 | 0.839 | 0.309 | 0.100 |
| 8 6 2 8 | >2.000 | 0.625 | 0.178 | 0.851 | 0.346 | 0.121 |
| 8 6 7 7 | 0.295 | 0.096 | 0.019 | 0.024 | 0.016 | 0.014 |
| 8 9 1 3 | 1.207 | 0.400 | 0.082 | 0.527 | 0.241 | 0.098 |
| 8 9 1 9 | 0.126 | 0.066 | 0.023 | 0.827 | 0.454 | 0.226 |
| Control | 0.020 | 0.016 | 0.013 | 0.039 | 0.020 | 0.012 |

Example 4

Plastic beads with a diameter of 1/4 inch were treated with a solution of the monoclonal antibody MAb8513 of the present invention with a concentration of 10 µg/ml at 4°C overnight so as to fix the antibody to the plastic beads. After washing the beads with PBS, the beads were blocked with 1% BSA solution in PBS at 37°C for 24 hours. To a reaction tray, 50 µl of a serum sample, 150 µ l of 100 mM phosphate buffer (pH 6.0) containing 1M NaCl and the beads treated as above were added so as to allow the reaction at 45°C for 2 hours. The samples tested were collected from 27 patients suffering from ovarian cancer, 53 patients suffering from benign ovarian tumor and 37 normal persons. After the reaction, the beads were washed three times with PBS and 200 µl of MAb8628-HRP diluted 2000-fold with 20 mM phosphate buffer (pH 7.3) containing 1 wt% BSA and 1M NaCl was added so as to allow the reaction at 37°C for 1 hour. After the reaction, the beads were washed 4 times with PBS and were transferred to a test tube containing 300 µl of o-phenylenediamine solution with a concentration of 3 mg/ml. The coloring reaction was allowed to occur at room temperature for 30 minutes. After the reaction, 1 ml of 1N sulfuric acid was added to the test tube and the absorbance was measured at 492 nm. The enzyme activity was measured based on a preliminarily prepared calibration curve. The results are shown in Fig. 1.

As can be seen from Fig. 1, it was confirmed that diagnosis of ovarian cancer can be carried out by using the monoclonal antibody of the present invention.

**Claims**

1. A monoclonal antibody which specifically reacts with a cancer-related human galactosyltransferase, said galactosyltransferase having a molecular weight of about 50,000 determined by SDS-polyacrylamide gel electrophoresis under reduced condition, said galactosyltransferase having a reactivity with MAb4880 (FERM BP-1758) under native conditions, a part of said galactosyltransferase self-associating, the reactivity of said galactosyltransferase with MAb4880 and the degree of self-association of said galactosyltransferase being promoted when said galactosyltransferase is heated under reducing conditions so that a part of said galactosyltransferase being detected in a fraction of a molecular weight of not less than 200,000 in gel permeation chromatography.

2. The monoclonal antibody of claim 1, which is MAb7907, MAb8513 or MAb8677.

3. A hybridoma producing said monoclonal antibody claimed in claim 1.

4. The hybridoma of claim 3, which is hybridoma 7907 (FERM BP-3279), hybridoma 8513 (FERM BP-3278) or hybridoma 8677 (FERM BP-3281).

5. A method of measuring cancer-related human galactosyltransferase in a sample comprising specifically reacting said monoclonal antibody of claim 1 with the cancer-related human galactosyltransferase possibly contained in said sample.

6. The method of claim 5, wherein said monoclonal antibody is MAb7907, MAb8513 or MAb8677.

FIG. 1

| DOCUMENTS CONSIDERED TO BE RELEVANT | EP 91103351.2 |

| Category | Citation of document with indication, where appropriate, of relevant passages | Relevant to claim | CLASSIFICATION OF THE APPLICATION (Int. Cl.5) |
|---|---|---|---|
| X | EP - A2/A3 - 0 226 888 (KONISHIROKU PHOTO INDUSTRY CO. LTD.) * Claims 1,4,6,9,11 * | 1-6 | C 12 P 21/08 C 12 Q 1/48 G 01 N 33/577 |
| X | CHEMICAL ABSTRACTS, vol. 111, No. 11, September 11, 1989, Columbus, Ohio, USA S. NOZAWA "The usefulness and limitation of sugar antigen in ovarian cancers. With special reference to a new tumor marker, CA 54/61" page 516, left column, abstract-No. 94 720v & Gan to Kagaku Ryoho 1989, 16(4, Pt. 2/1), 1147-51 | 1-6 | |
| X | CHEMICAL ABSTRACTS, vol. 102, No. 1, January 7, 1985, Columbus, Ohio, USA S.K. CHATTER JEE et al. "Murine monoclonal antibodies against galactosyl transferase from the ascites of ovarian cancer patients" page 410, left column, abstract-No. 4 295x & Cancer Res. 1984,44 (12, Pt.1), 5725-32 | 1-4 | TECHNICAL FIELDS SEARCHED (Int. Cl.5) C 12 P C 12 Q G 01 N |
| P,A | EP - A1 - 0 378 188 (KONICA CORPORATION) * Totality * | 1,5,6 | |
| A | CHEMICAL ABSTRACTS, vol. 110, No. 21, May 22, 1989, Columbus, Ohio, USA M. UEMURA et al. "Antiioliotypic antibody to galactosyl | 1,5 | |

The present search report has been drawn up for all claims

| Place of search | Date of completion of the search | Examiner |
|---|---|---|
| VIENNA | 10-06-1991 | AUGUSTIN |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | transferase for immunoassay and cancer diagnosis" page 597, left column, abstract-No. 191 089h & Jpn. Kokai Tokkyo Koho JP 63,214,669 (88,214,669) (Cl. G01N 33/573), 07 Sep 1988, Appl. 87/49,148, 03 Mar 1987 ---- | | |

TECHNICAL FIELDS  
SEARCHED (Int. Cl.5)

The present search report has been drawn up for all claims

| Place of search | Date of completion of the search | Examiner |
|---|---|---|
| VIENNA | 10-06-1991 | AUGUSTIN |

CATEGORY OF CITED DOCUMENTS

X : particularly relevant if taken alone  
Y : particularly relevant if combined with another document of the same category  
A : technological background  
O : non-written disclosure  
P : intermediate document

T : theory or principle underlying the invention  
E : earlier patent document, but published on, or after the filing date  
D : document cited in the application  
L : document cited for other reasons

  .......................................................................

& : member of the same patent family, corresponding document

EPO FORM 1503 03.82 (P0401)